# EUROPEAN PATENT APPLICATION

(11) **EP 0 724 875 A1**
(43) Date of publication of application: **07.08.1996**
(21) Application number: 95107577.9
(22) Date of filing: 18.05.1995
(51) Int. Cl.: A61K 7/50

(54) **Emulsifying agents for cosmetic compositions**

(30) Priority: 01.02.1995 IT MI950171
(71) Applicant: 3V SIGMA S.p.A, I-20121 Milano (IT)
(72) Inventor: Quintini, Massimo, I-24100 Bergamo (IT); Barzaghi, Massimo, I-20052 Monza, (Milano) (IT)
(74) Representative: Minoja, Fabrizio

(57) **Abstract**

Copolymers of unsaturated carboxylic acid with vinyl esters of branched chain aliphatic acids containing up to 10 carbon atoms, having formula (I), 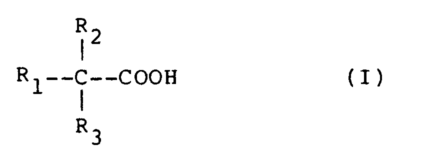
wherein R₁, R₂, R₃ are straight alkyl groups, are described together with a method of the use thereof as emulsifying agents in cosmetic compositions.

## Description

The present invention relates to cosmetic compositions, in particular to emulsifying agents for cosmetic compositions.

### BACKGROUND OF THE INVENTION

Cosmetic compositions are very complex formulations which comprise a high number of components having different physico-chemical characteristics. Often cosmetic compositions are made of multiphase systems not easily miscible one with the other, for example an agueous phase with an oily phase.

According to a common practice, particular substances are used to obtain an homogeneous mixture, these are the emulsifying agents, necessary to obtain a homogeneous composition.

Copolymers of carboxylic acids containing an olefin bond, as for example acrylic and methacrylic acids, with vinyl esters of branched chain aliphatic acids, hereinafter also named trialkylacetic acids, having formula (I) 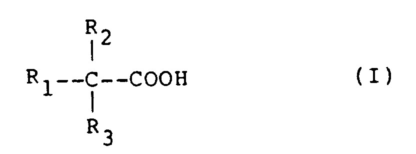
wherein R₁, R₂ and R₃, which can be the same or different each other, are straight alkyl groups, and the sum of the carbon atoms of the acid (I), the carboxylic group one included, is from 5 to 10, these copolymers being optionally crosslinked with other unsaturated monomers, are well-known in the art, see for example Italian patent n. 1,200,111.

Said copolymers, after neutralization of the free acid groups, are capable of giving a high viscosity to the polar solvents which they are incorporated in.

The phenomenon is particularly evident in the case of water, which is the solvent of choice for these salified copolymers, but it occurs also with polar organic solvents, such as for example methyl alcohol, ethyl alcohol, dimethylformamide.

A specific characteristic of these copolymers is not only to give a high viscosity to the solutions containing them, but also to maintain this high viscosity also if electrolytes are dissolved in the solvent medium, such as for example sodium chlorides or sulfates, ammonium sulfate or phosphate, dyes and anionic surfactants. For this reason, they find their application especially in those technological fields wherein dispersions or solutions containing electrolytes, such as for example textile printing pastes or liquid detergents, are to be used.

### SUMMARY OF THE INVENTION

It has now surprisingly been found that the above described copolymers can be used in cosmetic formulations or compositions, apart from as thickening agents or viscosity controllers, also in total or partial substitution of the conventionally used emulsifying agents in this field, such as for example the well-known marketed products with the Trade Names Tween^{(R)}, Span^{(R)} or glyceryl monostearate, fatty acid soaps, ethoxylated oleic and cetyl alcohols, ethoxylated lanolin, lecithin and other else.

In this way the formulation and the preparation of the compositions is simpler.

Moreover, the so obtained compositions give the skin subjected to the cosmetic treatment a non-soapy particularly smooth and velvety touch, not obtainable with conventional surfactants.

While the applicants wish to be unbound to any particular theoretical consideration, they deem this effect is achieved because the obtained emulsion breaks when contacted with skin, releasing the emulsified phase without emulsioning cutaneous fat.

It is an object of the present invention a method of using the above described copolymers as emulsifying agents in cosmetic compositions in partial or total substitution of the conventional emulsifying agents.

Further objects of the present invention are a process for the preparation of cosmetic compositions, cosmetic compositions not containing conventional emulsifying agents or containing an amount of conventional emulsifying agents lower than the conventional amount, containing said copolymers as emulsifying agents.

### DETAILED DISCLOSURE OF THE INVENTION

According to the present invention, the copolymers consist of:
a) from 98 to 80% of one or more carboxylic acids containing an olefinic double bond;
b) from 2 to 20% of one or more vinyl esters of trialkylacetic acids having formula (I) 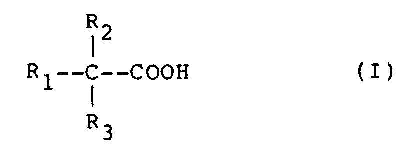 wherein R₁, R₂ and R₃, which can be the same or different each other; are straight alkyl groups, and the sum of the carbon atoms of the acid (I), the carboxylic group one included, is comprised between 5 and 10; and, optionally,
c) from 0 to 3%, calculated on the sum of a) and b), of one or more unsaturated monomers as crosslinking agents.

The preparation methods and the different compositions of the copolymer are described in the above mentioned Italian Patent 1,200,111.

To the purpose of the present invention, the copolymers in which the component a) is acrylic acid, particularly those in which the crosslinking agent is selected from the group consisting in pentaerhytritol, saccharose or trimethylolpropane allyl ether, are preferred.

According to the present invention, the emulsifying agents are used in amounts ranging from 0.01 and 10%, preferably from 0.1 and 1% by weight with respect to the total weight of the composition.

According to the present invention it is therefore possible to make stable emulsions both of the oil-in-water kind and of the water-in-oil kind. Therefore, other objects of the present invention are a method of forming stable emulsions by using the above copolymers as emulsifying agents in general.

The emulsifying agents can be used in cosmetic compositions in the form of water-in-oil (w/o) emulsions, as well as oil-in-water (o/w) emulsions.

Examples of said compositions are creams, milks, lotions, such as day- or night-creams, sun-creams, moisturizing creams and milks.

Typical ingredients of these compositions are triglycerides of vegetal or animal origin, mineral oils such as petroleum jelly and paraffin, natural waxes, fatty acid esters, fatty alcohols and acids, silicones and the like.

The amounts of the copolymers of the present invention for use in the compositions have a wide range, depending on the nature and amount of the components to be emulsified in the cosmetics. They are comprised between 0.01% and 10%, preferably between 0.1% and 1%.

With regard to the practical use of these copolymers, they must be predispersed in one of the two phases, that are present in the cosmetic composition, namely the aqueous phase or the oily phase, which is then mixed with the other phase, namely the oily phase or the aqueous phase, finally, pH is adjusted to the desired value with alkaline substances, as for example sodium hydroxide, potassium hydroxide, alkanolamine, fatty amines, aminomethylpropanol, aminoethylpropandiol, tris(hydroxyethyl)aminomethane, and the like.

The process for the preparation of cosmetic compositions, as well as the method to obtain an water-in-oil or oil-in-water emulsion are a further object of the present invention.

The examples which follow illustrate the preparative manner and the characteristics of some cosmetic compositions prepared with the copolymers object of the present invention in comparison with the same compositions containing conventional emulsifying agents. The copolymers of the examples were prepared according to the methods described in the Italian Patent n. 1,200,111 and, more precisely:
copolymer A is a copolymer of acrylic acid with the vinyl ester of an acid of formula (I) having 10 carbon atoms in total, in the 90:10 weight ratio and crosslinked with 0.5%, calculated on the weight of the monomers, of pentaerhytritol triallyl ether;
copolymer B is a copolymer of acrylic acid with the vinyl ester of an acid of formula (I) having 8 carbon atoms in total, in the 95:5 weight ratio and crosslinked with 1%, calculated on the weight of the monomers, of triallyl ether of trimethylolpropane;
copolymer C is a copolymer of acrylic acid with the vinyl ester of an acid of formula (I) having 5 carbon atoms in total, in 85:15 weight ratio and crosslinked with 0.3%, calculated on the weight of the monomers, of hexaallyl ether of saccharose.

### Example 1 - Night-cream

| Composition of the water-phase: | | |
|---|---|---|
| | according to the art | according to the invention |
| water | 77.8 | 81.8 |
| imidazolidinyl urea | 0.3 | 0.3 |
| methylparaben | 0.1 | 0.1 |
| Carbomer 934 | 0.3 | - |
| Copolymer A | - | 0.3 |
| glycerine | 3.0 | 3.0 |

| Composition of the oil-phase: | | |
|---|---|---|
| | according to the art | according to the invention |
| mineral oil | 5.0 | 5.0 |
| isopropyl myristate | 6.0 | 6.0 |
| cetyl alcohol | 3.0 | 3.0 |
| polysorbate 20 (emulsifier) | 2.0 | - |
| glyceryl stearate (emulsifier) | 2.0 | - |
| propylparaben | 0.05 | 0.05 |

Both the water-phase and the oil-phase were warmed to 70°C, then, under a good stirring, the oil-phase was added to the water-phase then acidity was neutralized with 0.45 parts of triethanolamine.

Both the creams appeared as well spreadable homogeneous pastes. However, the cream prepared according to the invention gave the skin a very velvety and more pleasant touch than the one obtainable with the cream prepared according to the art.

The same pleasant result for the skin, as well as an excellent stability of the emulsions, was obtained with the compositions of the examples which follow.

### Example 2 - Softening cream

| Composition of the water-phase: | | |
|---|---|---|
| | according to the art | according to the invention |
| water | 70.65 | 74.65 |
| imidazolidinyl urea | 0.3 | 0.3 |
| methylparaben | 0.1 | 0.1 |
| Carbomer 934 | 0.3 | - |
| Copolymer A | - | 0.3 |
| allantoin | 0.15 | 0.15 |
| hydrolyzed animal protein | 2.0 | 2.0 |

| Composition of the oil-phase: | | |
|---|---|---|
| | according to the art | according to the invention |
| hydroxylated lanolin | 3.0 | 3.0 |
| hydrogenated castor oil | 3.0 | 3.0 |
| cetyl alcohol | 4.0 | 4.0 |
| isopropyl palmitate | 6.0 | 6.0 |
| octyl dodecanol | 6.0 | 6.0 |
| polysorbate 20 (emulsifier) | 2.0 | - |
| ceteareth 20 (emulsifier) | 2.0 | - |
| propylparaben | 0.05 | 0.05 |

The two phases were mixed according to the procedure described in Example 1 and neutralized with 0.45 parts of triethanolamine.

### Example 3 - Moisturizing cream

| Composition of the water-phase: | | |
|---|---|---|
| | according to the art | according to the invention |
| water | 71.05 | 75.05 |
| imidazolidinyl urea | 0.3 | 0.3 |
| methylparaben | 0.1 | 0.1 |
| Carbomer 941 | 0.4 | 0.2 |
| Copolymer B | - | 0.2 |
| propylene glycol | 5.0 | 5.0 |

| Composition of the oil-phase: | | |
|---|---|---|
| | according to the art | according to the invention |
| caprilic triglyceride | 7.0 | 7.0 |
| ethoxylated nonylphenol (emulsifier) | 1.0 | - |
| PEG 4 octanoate (emulsifier) | 1.0 | - |
| cetyl alcohol | 3.0 | 3.0 |
| lanolin | 0.5 | 0.5 |
| octyl dodecanol | 5.0 | 5.0 |
| polysorbate 60 (emulsifier) | 2.0 | - |
| octyl stearate | 3.0 | 3.0 |
| propylparaben | 0.05 | 0.05 |

The two phases were mixed according to the procedure described in Example 1 and neutralized with 0.60 parts of triethanolamine.

### Example 4 - Body lotion

| Composition of the water-phase: | | |
|---|---|---|
| | according to the art | according to the invention |
| water | 79.55 | 84.55 |
| glycerine | 3.0 | 3.0 |
| imidazolidinyl urea | 0.3 | 0.3 |
| methyl paraben | 0.1 | 0.1 |

| Composition of the oil-phase: | | |
|---|---|---|
| | according to the art | according to the invention |
| stearic acid (emulsifier) | 2.0 | - |
| acetylated lanolin | 0.5 | 0.5 |
| mineral oil | 10.0 | 10.0 |
| cetyl alcohol | 0.5 | 0.5 |
| bee wax | 0.5 | 0.5 |
| ceteareth 20 (emulsifier) | 2.0 | - |
| Carbomer 941 | 0.2 | - |
| Copolymer C | - | 0.2 |

Copolymer C and Carbomer were dispersed in the oil-phase at 55°C under stirring. The oil-phase was subsequently added, while stirring, to the water-phase, pre-warmed at 55°C. The composition according to the art was neutralized with 1.3 parts of triethanolamine and the one according to the invention with only 0.3 parts (because stearic acid was absent).

### Example 5 - Protective cream

| Composition of the water-phase: | | |
|---|---|---|
| | according to the art | according to the invention |
| water | 59.98 | 63.08 |
| propylene glycol | 10.0 | 10.0 |
| imidazolidinyl urea | 0.3 | 0.3 |
| methylparaben | 0.1 | 0.1 |

| Composition of the oil-phase: | | |
|---|---|---|
| | according to the art | according to the invention |
| octyldodecyl/stearoyl stearate | 3.0 | 3.0 |
| acid of lanolin | 5.0 | 5.0 |
| isopropyl lanolate | 2.0 | 2.0 |
| isopropyl myristate | 5.0 | 5.0 |
| bee wax | 0.5 | 0.5 |
| mineral oil | 10.0 | 10.0 |
| cholesterol | 0.2 | 0.2 |
| glyceryl stearate (emulsifier) | 2.0 | - |
| polysorbate 61 (emulsifier) | 2.0 | - |
| propylparaben | 0.05 | 0.05 |
| Carbomer 941 | 0.2 | 0.2 |
| Copolymer A | - | 0.2 |

Copolymer A and Carbomer 941 were dispersed in the oil-phase at 55°C under stirring; the water-phase, warmed at 55°C, was subsequently added to the oil-phase while stirring. The composition was neutralized with 0.27 parts of 30% sodium hydroxide.

### Example 6 - Sun-lotion

| Composition of the water-phase: | | |
|---|---|---|
| | according to the art | according to the invention |
| water | 71.20 | 77.20 |
| imidazolidinyl urea | 0.3 | 0.3 |
| methylparaben | 0.1 | 0.1 |
| Carbomer 934 | 0.3 | - |
| Copolymer A | - | 0.3 |
| allantoin | 0.1 | 0.1 |

| Composition of the oil-phase: | | |
|---|---|---|
| | according to the art | according to the invention |
| acetylated lanolin | 1.0 | 1.0 |
| cetyl alcohol | 2.0 | 2.0 |
| isopropyl myristate | 4.0 | 4.0 |
| polysorbate 20 (emulsifier) | 2.0 | - |
| ceteareth 12 (emulsifier) | 2.0 | - |
| gliceryl stearate (emulsifier) | 2.0 | - |
| diisopropyl adipate | 0.5 | 0.5 |
| dioctyl cyclohexane | 3.0 | 3.0 |
| mineral oil | 4.0 | 4.0 |
| propylparaben | 0.05 | 0.05 |
| octyl-methoxy-cinnamate | 4.0 | 4.0 |
| benzophenone 3 | 3.0 | 3.0 |

Copolymer A and Carbomer were dispersed under stirring at 70°C in the water-phase; the oil-phase, pre-warmed to 70°C, was subsequently added thereto, while stirring. The resulting composition was neutralized with 0.28 parts of aminomethylpropanol.

## Claims

1. A process for the preparation of cosmetic compositions in the form of oil-in-water or water-in-oil emulsions, said compositions not containing conventional emulsifying agents or containing an amount of conventional emulsifying agents lower than conventional amount, said process comprising the use as emulsifying agent of at least one copolymer consisting of:
a) from 98 to 80% of one or more carboxylic acids containing an olefinic double bond;
b) from 2 to 20% of one or more vinyl esters of trialkylacetic acids having formula (I) 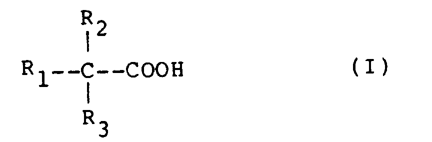 wherein R₁, R₂ and R₃, which can be the same or different each other, are straight alkyl groups and the sum of the carbon atoms, the carboxylic group one included, is between 5 and 10;
c) from 0 to 3%, calculated on the sum of a) and b), of one or more unsaturated monomers,
and subsequent neutralization.

2. A process according to claim 1, wherein acrylic acid is the carboxylic acid containing a double bond a).

3. A process according to claim 1, wherein the unsaturated monomer c) is selected from the group consisting of pentaerhytritol, saccharose or trimethylolpropane allyl ethers.

4. A process according to claims 1-3, wherein the emulsion to be formed is a water-in-oil type.

5. A process according to claims 1-3, wherein the emulsion to be formed is an oil-in-water type.

6. A process according to claims 1-5, wherein an amount of copolymer ranging from 0.01 to 10% by weight with respect to weight of the composition, is used.

7. A process according to claim 6, wherein an amount of copolymer ranging from 0.1 to 1% by weight with respect to weight of the composition, is used.

8. A process according to claims 1-7, for the preparation of creams, milks and lotions.

9. A method for forming a oil-in-water or water-in-oil emulsion comprising the use of one or more emulsifying agents according to claims 1-7.

10. Cosmetic compositions in the form of emulsions, not containing conventional emulsifying agents or containing an amount of conventional emulsifying agents lower than conventional amount, containing as emulsifying agent at least one copolymer consisting of:
a) from 98 to 80% of one or more carboxylic acids containing an olefinic double bond ;
b) from 2 to 20% of one or more vinyl esters of trialkylacetic acids having formula (I) 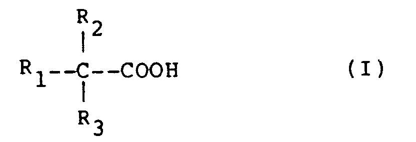 wherein R₁, R₂ and R₃, which can be the same or different each other, are straight alkyl groups and the sum of carbon atoms of the acid (I), the carboxylic group one included, is between 5 and 10;
c) from 0 to 3%, calculated on the sum of a) and b), of one or more unsaturated monomers.

11. Compositions according to claim 10, containing from 0.01 to 10% by weight with respect to the weight of the composition, of said copolymer.

12. Compositions according to claim 11, containing from 0.1% to 1% by weight, with respect to the weight of the composition, of said copolymer.

13. Compositions according to claims 10-12, in the form of creams, milks, sun-lotions.

14. Compositions according to claims 10-12, selected from the group consisting of day- or night-creams, softening cream, moisturizing cream, body lotion, protective cream, sun-lotion.
